Europäisches Patentamt

European Patent Office

Office européen des brevets

(11) Publication number: **0 475 311 A1**

# EUROPEAN PATENT APPLICATION

(21) Application number: **91115160.3**

(22) Date of filing: **07.09.91**

(51) Int. Cl.5: **C07K 3/18**, C12N 15/03, C12N 1/21, //(C12N1/21, C12R1:19)

(30) Priority: **14.09.90 JP 242651/90**

(43) Date of publication of application: **18.03.92 Bulletin 92/12**

(84) Designated Contracting States: **DE ES FR GB IT**

(71) Applicant: **Nippon Kayaku Kabushiki Kaisha 11-2, Fujimi-cho 1 chome Chiyoda-ku Tokyo(JP)**

(72) Inventor: **Kajihara, Junichi 6-2-105-101, Kaminotani, Suma-ku Koube-shi, Hyogo-ken(JP)**
Inventor: **Takagi, Kenichi 7-19-22, Ouizumigakuen-chou Nerima-ku, Tokyo(JP)**

(74) Representative: **Türk, Gille, Hrabal Brucknerstrasse 20 W-4000 Düsseldorf 13(DE)**

(54) Process for removing proteins originating from Escherichia coli.

(57) A process for separating and purifying a recombinant protein from proteins originating from Escherichia coli is disclosed.

The process of the present invention comprises dialyzing a solution, which contains a useful protein such as recombinant human Cu-Zn superoxide dismutase (r-h Cu-Zn SOD) or catalase together with proteins originating from Escherichia coli, against a buffer solution the pH value of which has been controlled by adjusting the salt concentration and using buffer components, contacting the solution with a phenylated resin which has been equilibrated with said buffer solution and thus adsorbing the proteins originating from Escherichia coli while passing the useful protein therethrough to thereby remove the proteins originating from Escherichia coli.

EP 0 475 311 A1

Background of the Invention:

There has been required a process whereby proteins originating from Escherichia coli can be efficiently removed from an aqueous solution of, for example, a recombinant protein produced by using Escherichia coli which is most commonly employed as a host in genetic recombination.

In recent years, the use of physiologically active proteins produced on a mass scale through genetic engineering techniques as drugs has rapidly increased. However there is a problem from the viewpoint of safety that such a physiologically active protein might be contaminated with components originating from a microorganism employed as a host, in particular, somatic proteins, unlike conventional ones obtained by extracting living tissues or the like. Thus it is required to minimize the contamination with the substances originating from microorganisms. However known principal methods for efficiently removing these contaminants require highly complicated procedures, for example, a combination of various column chromatographic techniques.

Summary of the Invention:

In order to solve these problems, the present inventors have conducted intensive studies to find out that the proteins originating from Escherichia coli can be selectively adsorbed by a phenylated resin and thus efficiently removed from the target protein fraction, thus completing the present invention.

Namely, a recombinant protein can be obtained at a high purification yield by a single procedure wherein the equilibration of a resin, the contact of a protein originating from Escherichia coli with said resin and adsorption thereby and the passage of the target recombinant protein are effected by using a single buffer solution. The process of the present invention is convenient and advantageous from an industrial viewpoint.

Detailed Description of the Invention:

This invention relates to a process for removing proteins originating from Escherichia coli from an aqueous solution containing proteins originating from Escherichia coli together with a useful protein, which comprises contacting said aqueous solution with a phenylated resin and thus adsorbing said proteins originating from Escherichia coli with said resin while passing said useful protein therethrough.

Any aqueous solution containing proteins originating from Escherichia coli may be used in the present invention so long as it contains substances originating from Escherichia coli as impurities. A common example thereof is an aqueous solution containing a protein produced by a genetic recombination method with the use of Escherichia coli as a host. Such an aqueous solution of a recombinant protein includes a supernatant obtained after grinding cells and partially purified ones, but a roughly purified one is efficiently treated by the process of the present invention.

When the aqueous solution containing proteins originating from Escherichia coli is an aqueous solution of a useful protein, as the above-mentioned aqueous solution of a recombinant protein, it is necessary to control, for example, the pH value and salt concentration of the aqueous solution so as to allow said phenylated resin to adsorb not the useful protein but the proteins originating from Escherichia coli selectively.

The recombinant protein contained in the aqueous solution is not particularly restricted. Examples thereof include superoxide dismutase (SOD) and catalase. Recently, SOD, in particular, human Cu-Zn SOD which is expected as a remedy for ischemic reperfusion disorders and inflammation, has been produced by genetic recombination techniques. The process of the present invention is suitable for purifying this SOD.

As the phenylated resin to be used in the present invention, those wherein phenyl group is bound to an uncharged carrier, for example, agarose, hydrophilic vinyl polymer or cellulose, may be used. Examples thereof include Phenyl Sepharose® (product of Pharmacia AB), Phenyl Toyopearl® (product of Tosoh Co.) and Phenyl Cellulofine® (product of Seikagaku Kogyo K.K.). Further, HPLC with the use of these carriers as a base can be used in the same manner as that for an open column and thus achieve a satisfactory effect. Among these resins, phenylated cellulose is preferable.

In the process of the present invention, 0.2 to 2.0 M, preferably 0.5 to 1.5 M and still preferably 0.7 to 1.3 M, of ammonium sulfate or a salt comparable to ammonium sulfate in effects (for example, NaCl, KCl or ammonium chloride) is added to a sample to be contacted with the phenylated resin. Then the pH value thereof is adjusted to 4 to 10, preferably 6 to 9 and still preferably 7.5 to 8.5, with a buffer component, for example, acetate such as sodium acetate or ammonium acetate, citrate such as sodium citrate or ammonium citrate, phosphate such as sodium phosphate or potassium phosphate or hydrochloride of organic amine such as triethanolamine or tris(hydroxymethyl)aminomethane, to thereby give a buffer solution. Thus the optimum conditions for selectively adsorbing not any recombinant protein but proteins originating from microorganisms can be achieved. The concentration of the buffer component to be added for adjusting the pH value is not particularly restricted so long as the pH value falls within the range as specified above. This concentration may range generally from 5 mM to 200 mM, preferably from 15 mM to 50 mM.

The content of the proteins originating from Escherichia coli in the buffer solution to be contacted with the resin may be below about 20,000 mg/l, while the content of the recombinant protein, for example, human Cu-Zn SOD therein may range from $4 \times 10^6$ U/l to $1.2 \times 10^9$ U/l.

The process of the present invention may be effected, for example, in the following manner. The phenylated resin is equilibrated with the above-mentioned buffer solution. Then the buffer solution of the same composition, in which the recombinant protein is dissolved together with proteins originating from Escherichia coli, is passed through the phenylated resin. Next, the phenylated resin is washed with 3 to 5 times as much the same buffer solution as the one used for equilibrating it. Thus the recombinant protein is recovered in the washing liquor at a high yield. Subsequently, the salt concentration is lowered to elute the proteins originating from Escherichia coli. Therefore the phenylated resin can be repeatedly used.

The recombinant protein obtained by the process of the present invention has a high purity and the proteins originating from Escherichia coli have been efficiently removed therefrom.

To further illustrate the present invention, and not by way of limitation, the following Examples will be given.

Example 1

10 ml of Phenyl Sepharose was equilibrated by passing 50 ml of a 20 mM tris hydrochloride buffer solution (pH: 8.0) containing 1 M of ammonium sulfate therethrough.

24 ml of a crude solution containing 7,000,000 U of human Cu-Zn superoxide dismutase (hereinafter abbreviated to r-hSOD) produced by Escherichia coli was dialyzed against the above-mentioned buffer solution. 20 ml of a buffer solution containing 7,000,000 U of r-hSOD and 63 mg of proteins originating from Escherichia coli was passed through the column and then washed with 50 ml of the same buffer solution. The recovery of the activity of the r-hSOD recovered in the washing liquor was 90% and the specific activity thereof was 4,000 U/mg of protein. When subjected to SDS electrophoresis under reductive conditions, the r-hSOD showed a single band of 16 K daltons.

As Table 1 shows, the content of the proteins originating from Escherichia coli was reduced below 1/36,000 of that prior to the purification with this column. In this Example, the SOD activity was determined by a method reported by McCord and Fridorich [J. Biol. Chem., 244, 6049 (1969)] while the proteins were determined by a method of Lowry et al. [J. Biol. Chem., 193, 265 (1951)]. The proteins originating from Escherichia coli were determined by enzyme immunoassay with the use of a polyclonal antibody obtained by immunizing Escherichia coli components.

Table 1

| | SOD (U) | Proteins originating from Escherichia coli (mg) |
|---|---|---|
| Before column passage | $7.0 \times 10^6$ (100%) | 63 (100%) |
| After column passage | $6.3 \times 10^6$ (90%) | $<1.75 \times 10^{-3}$ ($<2.8 \times 10^{-3}$%) |

Example 2

10 ml of Phenyl Sepharose was equilibrated by passing 50 ml of a 0.1 M sodium phosphate buffer solution (pH: 7.0) containing 1 M of ammonium sulfate therethrough.

10 ml of an Escherichia coli extract was added to 10 ml of a solution containing 400,000 U of catalase purified from human erythrocytes followed by mixing. Then it was dialyzed against the above-mentioned buffer solution. 17 ml of a buffer solution containing 400,000 U of catalase and 100 mg of proteins originating from Escherichia coli was passed through the column and then washed with 50 ml of the same buffer solution. Thus catalase was eluted in the washing liquor at an activity recovery of 95%. The content of the proteins originating from Escherichia coli was reduced below 1/50,000 of that prior to the purification with this column.

In this Example, the catalase activity was determined by a method reported by Beers and Sizer [J. Biol. Chem., 195, 133 (1952)] while the proteins were determined by the same methods as those employed in Example 1. Table 2 summarizes the results.

Table 2

|  | Catalase (U) | Proteins originating from Escherichia coli (mg) |
|---|---|---|
| Before column passage | $4.0 \times 10^5$ (100%) | 100 (100%) |
| After column passage | $3.8 \times 10^5$ (95%) | $<2.0 \times 10^{-3}$ ($<2.0 \times 10^{-3}$%) |

In each of the cases of the recombinant Cu-Zn h SOD and the catalase, the content of the somatic protein impurities was remarkably reduced by a single treatment and thus desirable results were obtained by the process of the present invention. Thus it has been proved that the process of the present invention, which is a convenient process with the use of a small amount of a resin, is commonly applicable to various proteins.

**Claims**

1. A process for removing proteins originating from Escherichia coli from an aqueous solution containing proteins originating from Escherichia coli as impurities together with a useful protein, which comprises contacting said aqueous solution with a phenylated resin and thus adsorbing said protein originating from Escherichia coli with said resin while passing said useful proteins therethrough.

2. A process as claimed in Claim 1, wherein said aqueous solution containing a useful protein together with proteins originating from Escherichia coli as impurities is a buffer solution containing a salt at a concentration of from 0.2 M to below 2 M and having a pH value of from 6 to 9.

3. A process as claimed in Claim 2, wherein said salt is ammonium sulfate.

4. A process as claimed in Claim 2, wherein said useful protein is a recombinant protein.

5. A process as claimed in Claim 4, wherein said recombinant protein is human Cu-Zn superoxide dismutase (r-h Cu-Zn SOD) or catalase.

6. A process as claimed in Claim 1, comprising passing a solution, which contains below 20,000 mg/l of

4

proteins originating from Escherichia coli and from $4 \times 10^6$ U/l to $1.2 \times 10^9$ U/l of r-h Cu-Zn SOD in a buffer solution containing from 0.7 to 1.3 M of ammonium sulfate and from 15 mM to 50 mM of tris hydrochloride and having a pH value of from 7.5 to 8.5, through a column packed with a phenylated cellulose resin, which has been equilibrated with said buffer solution, and thus adsorbing the proteins originating from Escherichia coli with said resin while passing r-h Cu-Zn SOD therethrough.

| DOCUMENTS CONSIDERED TO BE RELEVANT | | | EP 91115160.3 |
|---|---|---|---|
| Category | Citation of document with indication, where appropriate, of relevant passages | Relevant to claim | CLASSIFICATION OF THE APPLICATION (Int. Cl.5) |
| A | PATENT ABSTRACTS OF JAPAN, unexamined applications, C field, vol. 13, no. 293, July 6, 1989 THE PATENT OFFICE JAPANESE GOVERNMENT page 33 C 615 * Kokai-no. 1-86 871 (AGENCY OF IND SCIENCE & TECHNOL) * -- | 1-4 | C 07 K 3/18 C 12 N 15/03 C 12 N 1/21 //(C 12 N 1/21 C 12 R 1:19) |
| A | CHEMICAL ABSTRACTS, vol. 109, no. 21, November 21, 1988, Columbus, Ohio, USA M. UHLEN et al. "Genetic design to facilitate recovery of bioproducts secretion in Escherichia coli and affinity purification" page 565, right column, abstract-no. 188 659t & World Biotech Rep. 1987, 1(Pt. 3), 15-23 -- | 1,4 | |
| A | EP - A2 - 0 333 691 (CEMU BIOTEKNIK AB) * Claims 14-17 * -- | 1,4 | TECHNICAL FIELDS SEARCHED (Int. Cl.5) C 07 K C 12 N |
| P,A | CHEMICAL ABSTRACTS, vol. 115, no. 1, July 8, 1991, Columbus, Ohio, USA H.V. LE et al. "Purification of secreted recombinant proteins from Eschichia coli" page 692, right column, abstract-no. 6 846v & Bioprocess Technol. 1991, 12(Purif. Anal. Recomb. | 1,4 | |

The present search report has been drawn up for all claims

| Place of search | Date of completion of the search | Examiner |
|---|---|---|
| VIENNA | 19-11-1991 | AUGUSTIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

| DOCUMENTS CONSIDERED TO BE RELEVANT | | | EP 91115160.3 | |
|---|---|---|---|---|
| Category | Citation of document with indication, where appropriate, of relevant passages | Relevant to claim | CLASSIFICATION OF THE APPLICATION (Int. Cl. 5) | |
| | Proteins), 163-81<br>-- | | | |
| A | CHEMICAL ABSTRACTS, vol. 106,<br>no. 19, May 11, 1987,<br>Columbus, Ohio, USA<br>T. ATKINSON et al.<br>"High-level microbial<br>expression and purification<br>of recombinant proteins"<br>page 544, left and right<br>column, abstract-no. 154 667s<br>& Spec. Publ. Soc. Gen.<br>Microbiol. 1986, 18(Bioact.<br>Microb. Prod. 3), 27-43<br>-- | 1,4 | | |
| A | CHEMICAL ABSTRACTS, vol. 105,<br>no. 25, December 22, 1986,<br>Columbus, Ohio, USA<br>F.A.O. MARSTON<br>"The purification of<br>eukaryotic polypeptides<br>synthesized in Escherichia<br>coli"<br>page 615, right column,<br>abstract-no. 224 478m<br>& Biochem. J. 1986, 240(1),<br>1-12<br>-- | 1,4 | TECHNICAL FIELDS SEARCHED (Int. Cl.5) | |
| A | PATENT ABSTRACTS OF JAPAN,<br>unexamined applications,<br>C field, vol. 12, no. 405,<br>October 26, 1988<br>THE PATENT OFFICE JAPANESE<br>GOVERNMENT<br>page 81 C 539<br>* Kokai-no. 63-145 297<br>(SAGAMI CHEM RES CENTER) *<br>---- | 1,4 | | |
| | The present search report has been drawn up for all claims | | | |
| | Place of search<br>VIENNA | Date of completion of the search<br>19-11-1991 | Examiner<br>AUGUSTIN | |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)